# EUROPEAN PATENT APPLICATION

(11) **EP 4 006 058 A1**
(43) Date of publication of application: **01.06.2022**
(21) Application number: 20844267.3
(22) Date of filing: 08.07.2020
(51) Int. Cl.: C07K 19/00, A61K 38/18, A61K 38/26, A61P 3/10

(54) **FGF21 FC FUSION PROTEIN, GLP-1 FC FUSION PROTEIN, AND COMBINATION THERAPEUTIC AGENT COMPRISING SAME AND USE THEREOF**

(30) Priority: 25.07.2019 CN 201910675288
(71) Applicant: Ampsource Biopharma Shanghai Inc., Shanghai 201318 (CN)
(72) Inventor: DONG, Zhao, Shanghai 201318 (CN); ZHOU, Chi, Shanghai 201318 (CN); ZHANG, Jiyu, Shanghai 201318 (CN); LI, Yuanli, Shanghai 201318 (CN); LI, Qiang, Shanghai 201318 (CN)
(74) Representative: HGF
(86) International application number: PCT/CN2020/100774
(87) International publication number: WO 2021/012947

(57) **Abstract**

An FGF21 Fc fusion protein, a GLP-1 Fc fusion protein, and a combination therapeutic agent. The combination therapeutic agent consists of a first pharmaceutical composition comprising an FGF21 Fc fusion protein and a second pharmaceutical composition comprising a GLP-1 Fc fusion protein. The fusion proteins or a combination thereof is used for preventing or curing cardiovascular diseases and/or metabolic diseases; the diseases comprise obesity, diabetes, hyperlipidemia, nonalcoholic fatty liver disease, atherosclerosis, diabetic cardiomyopathy, coronary atherosclerotic cardiomyopathy, and other diseases related to insulin resistance.

SEQUENCE LISTING

<110>  AMPSOURCE BIOPHARMA SHANGHAI INC.

<120>  FGF21 FC FUSION PROTEIN, GLP-1 FC FUSION PROTEIN, AND COMBINATION
       THERAPEUTIC AGENT COMPRISING SAME AND USE THEREOF

<130>  P337821EP / KRE

<140>  EP20844267.3
<141>  2020-07-08

<150>  PCT/CN2020/100774
<151>  2020-07-08

<150>  CN201910675288.5
<151>  2019-07-25

<160>  7

<170>  PatentIn version 3.5

<210>  1
<211>  209
<212>  PRT
<213>  Homo sapiens

<400>  1

Met Asp Ser Asp Glu Thr Gly Phe Glu His Ser Gly Leu Trp Val Ser
1               5                   10                  15

Val Leu Ala Gly Leu Leu Leu Gly Ala Cys Gln Ala His Pro Ile Pro
            20                  25                  30

Asp Ser Ser Pro Leu Leu Gln Phe Gly Gly Gln Val Arg Gln Arg Tyr
        35                  40                  45

Leu Tyr Thr Asp Asp Ala Gln Gln Thr Glu Ala His Leu Glu Ile Arg
    50                  55                  60

Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95

Lys Thr Ser Arg Phe Leu Cys Gln Arg Pro Asp Gly Ala Leu Tyr Gly
            100                 105                 110

Ser Leu His Phe Asp Pro Glu Ala Cys Ser Phe Arg Glu Leu Leu Leu
        115                 120                 125

Glu Asp Gly Tyr Asn Val Tyr Gln Ser Glu Ala His Gly Leu Pro Leu
    130                 135                 140

His Leu Pro Gly Asn Lys Ser Pro His Arg Asp Pro Ala Pro Arg Gly
145                 150                 155                 160

Pro Ala Arg Phe Leu Pro Leu Pro Gly Leu Pro Pro Ala Leu Pro Glu
                165                 170                 175

Pro Pro Gly Ile Leu Ala Pro Gln Pro Pro Asp Val Gly Ser Ser Asp
            180                 185                 190

Pro Leu Ser Met Val Gly Pro Ser Gln Gly Arg Ser Pro Ser Tyr Ala
        195                 200                 205

Ser

<210>  2
<211>  31
<212>  PRT
<213>  Homo sapiens

<400>  2

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  3
<211>  39
<212>  PRT
<213>  Heloderma suspectum

<400>  3

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  4
<211>  459
<212>  PRT
<213>  Artificial Sequence

<220>
<223>  FP4I amino acid sequence

<400>  4

His Pro Ile Pro Asp Ser Ser Pro Leu Leu Gln Phe Gly Gly Gln Val
1               5                   10                  15

Arg Gln Arg Tyr Leu Tyr Thr Asp Asp Ala Gln Gln Thr Glu Ala His
            20                  25                  30

Leu Glu Ile Arg Glu Asp Gly Thr Val Gly Gly Ala Ala Asp Gln Ser
        35                  40                  45

Pro Glu Ser Leu Leu Gln Leu Lys Ala Leu Lys Pro Gly Val Ile Gln
    50                  55                  60

Ile Leu Gly Val Lys Thr Ser Arg Phe Leu Cys Gln Arg Pro Asp Gly
65                  70                  75                  80

Ala Leu Tyr Gly Ser Leu His Phe Asp Pro Glu Ala Cys Ser Phe Arg
                85                  90                  95

Glu Leu Leu Leu Glu Asp Gly Tyr Asn Val Tyr Gln Ser Glu Ala His
            100                 105                 110

Gly Leu Pro Leu His Leu Pro Gly Asn Lys Ser Pro His Arg Asp Pro
        115                 120                 125

Ala Pro Arg Gly Pro Ala Arg Phe Leu Pro Leu Pro Gly Leu Pro Pro
    130                 135                 140

Ala Pro Pro Glu Pro Pro Gly Ile Leu Ala Pro Gln Pro Pro Asp Val
145                 150                 155                 160

Gly Ser Ser Asp Pro Leu Ser Met Val Gly Pro Ser Gln Gly Arg Ser
                165                 170                 175

Pro Ser Tyr Ala Ser Gly Ser Gly Gly Gly Gly Ser Gly Gly Gly Gly
            180                 185                 190

Ser Gly Gly Gly Gly Ser Gly Gly Gly Gly Ser Gly Gly Gly Gly Ser
        195                 200                 205

Ser Ser Ser Ser Lys Ala Pro Pro Pro Ser Leu Pro Ser Pro Ser Arg
    210                 215                 220

Leu Pro Gly Pro Ser Asp Thr Pro Ile Leu Pro Gln Val Glu Cys Pro
225                 230                 235                 240

Pro Cys Pro Ala Pro Pro Val Ala Gly Pro Ser Val Phe Leu Phe Pro
                245                 250                 255

Pro Lys Pro Lys Asp Gln Leu Met Ile Ser Arg Thr Pro Glu Val Thr
            260                 265                 270

Cys Val Val Val Asp Val Ser His Glu Asp Pro Glu Val Gln Phe Asn
        275                 280                 285

Trp Tyr Val Asp Gly Val Glu Val His Asn Ala Lys Thr Lys Pro Arg
    290                 295                 300

Glu Glu Gln Phe Asn Ser Thr Phe Arg Val Val Ser Val Leu Thr Val
305                 310                 315                 320

Val His Gln Asp Trp Leu Asn Gly Lys Glu Tyr Lys Cys Lys Val Ser
                325                 330                 335

Asn Lys Gly Leu Pro Ala Ser Ile Glu Lys Thr Ile Ser Lys Thr Lys
            340                 345                 350

Gly Gln Pro Arg Glu Pro Gln Val Tyr Thr Leu Pro Pro Ser Arg Glu
        355                 360                 365

Glu Met Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Met Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Leu His Glu Ala Leu His Asn His Tyr
        435                 440                 445

Thr Gln Lys Ser Leu Ser Leu Ser Pro Gly Lys
    450                 455

<210>  5
<211>  275
<212>  PRT
<213>  Artificial Sequence

<220>
<223>  Dulaglutide amino acid sequence

<400>  5

His Gly Glu Gly Thr Phe Thr Ser Asp Val Ser Ser Tyr Leu Glu Glu
1               5                   10                  15

Gln Ala Ala Lys Glu Phe Ile Ala Trp Leu Val Lys Gly Gly Gly Gly
            20                  25                  30

Gly Gly Gly Ser Gly Gly Gly Gly Ser Gly Gly Gly Gly Ser Ala Glu
        35                  40                  45

Ser Lys Tyr Gly Pro Pro Cys Pro Pro Cys Pro Ala Pro Glu Ala Ala
    50                  55                  60

Gly Gly Pro Ser Val Phe Leu Phe Pro Pro Lys Pro Lys Asp Thr Leu
65                  70                  75                  80

Met Ile Ser Arg Thr Pro Glu Val Thr Cys Val Val Val Asp Val Ser
                85                  90                  95

Gln Glu Asp Pro Glu Val Gln Phe Asn Trp Tyr Val Asp Gly Val Glu
            100                 105                 110

Val His Asn Ala Lys Thr Lys Pro Arg Glu Glu Gln Phe Asn Ser Thr
        115                 120                 125

Tyr Arg Val Val Ser Val Leu Thr Val Leu His Gln Asp Trp Leu Asn
    130                 135                 140

Gly Lys Glu Tyr Lys Cys Lys Val Ser Asn Lys Gly Leu Pro Ser Ser
145                 150                 155                 160

Ile Glu Lys Thr Ile Ser Lys Ala Lys Gly Gln Pro Arg Glu Pro Gln
                165                 170                 175

Val Tyr Thr Leu Pro Pro Ser Gln Glu Glu Met Thr Lys Asn Gln Val
            180                 185                 190

Ser Leu Thr Cys Leu Val Lys Gly Phe Tyr Pro Ser Asp Ile Ala Val
        195                 200                 205

Glu Trp Glu Ser Asn Gly Gln Pro Glu Asn Asn Tyr Lys Thr Thr Pro
    210                 215                 220

Pro Val Leu Asp Ser Asp Gly Ser Phe Phe Leu Tyr Ser Arg Leu Thr
225                 230                 235                 240

Val Asp Lys Ser Arg Trp Gln Glu Gly Asn Val Phe Ser Cys Ser Val
                245                 250                 255

Met His Glu Ala Leu His Asn His Tyr Thr Gln Lys Ser Leu Ser Leu
            260                 265                 270

Ser Leu Gly
        275

<210>  6
<211>  317
<212>  PRT
<213>  Artificial Sequence

<220>
<223>  FP-A amino acid sequence

<400>  6

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Gly Ser Gly Gly Gly Gly Ser Gly Gly
        35                  40                  45

Gly Gly Ser Gly Gly Gly Gly Ser Gly Gly Gly Gly Ser Gly Gly Gly
    50                  55                  60

Gly Ser Ser Ser Ser Ser Lys Ala Pro Pro Pro Ser Leu Pro Ser Pro
65                  70                  75                  80

Ser Arg Leu Pro Gly Pro Ser Asp Thr Pro Ile Leu Pro Gln Val Glu
                85                  90                  95

Cys Pro Pro Cys Pro Ala Pro Pro Val Ala Gly Pro Ser Val Phe Leu
            100                 105                 110

Phe Pro Pro Lys Pro Lys Asp Gln Leu Met Ile Ser Arg Thr Pro Glu
        115                 120                 125

Val Thr Cys Val Val Val Asp Val Ser His Glu Asp Pro Glu Val Gln
    130                 135                 140

Phe Asn Trp Tyr Val Asp Gly Val Glu Val His Asn Ala Lys Thr Lys
145                 150                 155                 160

Pro Arg Glu Glu Gln Phe Asn Ser Thr Phe Arg Val Val Ser Val Leu

165                 170                 175

Thr Val Val His Gln Asp Trp Leu Asn Gly Lys Glu Tyr Lys Cys Lys
            180                 185                 190

Val Ser Asn Lys Gly Leu Pro Ala Ser Ile Glu Lys Thr Ile Ser Lys
        195                 200                 205

Thr Lys Gly Gln Pro Arg Glu Pro Gln Val Tyr Thr Leu Pro Pro Ser
    210                 215                 220

Arg Glu Glu Met Thr Lys Asn Gln Val Ser Leu Thr Cys Leu Val Lys
225                 230                 235                 240

Gly Phe Tyr Pro Ser Asp Ile Ala Val Glu Trp Glu Ser Asn Gly Gln
                245                 250                 255

Pro Glu Asn Asn Tyr Lys Thr Thr Pro Pro Met Leu Asp Ser Asp Gly
            260                 265                 270

Ser Phe Phe Leu Tyr Ser Lys Leu Thr Val Asp Lys Ser Arg Trp Gln
        275                 280                 285

Gln Gly Asn Val Phe Ser Cys Ser Val Leu His Glu Ala Leu His Asn
    290                 295                 300

His Tyr Thr Gln Lys Ser Leu Ser Leu Ser Pro Gly Lys
305                 310                 315

<210>  7
<211>  278
<212>  PRT
<213>  Artificial Sequence

<220>
<223>  FP-B amino acid sequence

<400>  7

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Gly Ser Gly Gly Gly Ser Gly Gly Gly
        35                  40                  45

Gly Ser Gly Gly Gly Gly Ser Val Glu Cys Pro Pro Cys Pro Ala Pro
    50                  55                  60

Pro Val Ala Gly Pro Ser Val Phe Leu Phe Pro Pro Lys Pro Lys Asp
65                  70                  75                  80

Gln Leu Met Ile Ser Arg Thr Pro Glu Val Thr Cys Val Val Val Asp
                85                  90                  95

Val Ser His Glu Asp Pro Glu Val Gln Phe Asn Trp Tyr Val Asp Gly
            100                 105                 110

Val Glu Val His Asn Ala Lys Thr Lys Pro Arg Glu Glu Gln Phe Asn
        115                 120                 125

Ser Thr Phe Arg Val Val Ser Val Leu Thr Val Val His Gln Asp Trp
    130                 135                 140

Leu Asn Gly Lys Glu Tyr Lys Cys Lys Val Ser Asn Lys Gly Leu Pro
145                 150                 155                 160

Ala Ser Ile Glu Lys Thr Ile Ser Lys Thr Lys Gly Gln Pro Arg Glu
                165                 170                 175

Pro Gln Val Tyr Thr Leu Pro Pro Ser Arg Glu Glu Met Thr Lys Asn
            180                 185                 190

Gln Val Ser Leu Thr Cys Leu Val Lys Gly Phe Tyr Pro Ser Asp Ile
        195                 200                 205

Ala Val Glu Trp Glu Ser Asn Gly Gln Pro Glu Asn Asn Tyr Lys Thr
    210                 215                 220

Thr Pro Pro Met Leu Asp Ser Asp Gly Ser Phe Phe Leu Tyr Ser Lys
225                 230                 235                 240

Leu Thr Val Asp Lys Ser Arg Trp Gln Gln Gly Asn Val Phe Ser Cys
                245                 250                 255

Ser Val Leu His Glu Ala Leu His Asn His Tyr Thr Gln Lys Ser Leu
            260                 265                 270

Ser Leu Ser Pro Gly Lys
        275

## Description

### TECHNICAL FIELD

The present disclosure belongs to the technical field of proteins and polypeptides, and specifically, relates to an FGF21 Fc fusion protein, a GLP-1 Fc fusion protein, and a combination therapeutic agent including both for the prevention or treatment of cardiovascular diseases and/or metabolic diseases that include, but are not limited to, obesity, hyperlipidemia, atherosclerosis, non-alcoholic fatty liver disease, diabetes, diabetic cardiomyopathy, coronary atherosclerotic heart disease, and other diseases associated with insulin resistance.

### BACKGROUND

Fibroblast growth factor 21 (FGF21), a member of the FGF family, is mainly synthesized by the liver and then released into the circulation in the form of endocrine. The C terminus of FGF21 binds to the effector organ β-klotho transmembrane protein and then binds specifically to fibroblast growth factor receptor 1c (FGFR1c) through the N terminus to form a stable FGF21/β-klotho/FGFR complex and then activate downstream related molecular signals. A large number of fundamental researches have shown that FGF21 has many physiological activities such as promoting glucose utilization, increasing insulin sensitivity, promoting fatty acid decomposition, reducing lipid neogenesis, regulating cholesterol balance, etc., and shows great potential for application in cardiovascular diseases and metabolic diseases. However, natural FGF21 is prone to be filtered and metabolized by glomerulus and is also susceptible to hydrolytic cleavage under the action of protease, which leads to the short half-life *in vivo* and seriously restricts the druggability of natural FGF21. With the in-depth development of biotechnology, the structural modification of the natural FGF21 to prolong the half-life *in vivo* and improve the bioavailability has become a hotspot for research and development of major pharmaceutical companies worldwide. At present, various long-acting FGF21 proteins have undergone clinical studies, and most of them are subjected to polyethylene glycol modification, Fc protein fusion or CovX-Body covalent ligation to prolong the half-life of FGF21. Clinical studies have shown that the long-acting FGF21 protein has a positive therapeutic effect on the relief of hepatic steatosis in patients with non-alcoholic fatty liver disease, weight loss and blood lipid regulation, but fails to show significant pharmacodynamic advantages over the existing hypoglycemic drugs in the market in controlling blood glucose of patients with diabetes.

Compared with mammal (glucagon-like peptide-1) GLP-1 or Exendin-4, long-acting GLP-1 receptor agonists are a kind of protein products obtained by subjecting GLP-1 or Exendin-4 analogues to structural modification, which have greatly improved half-life and bioavailability *in vivo* and reduced injection frequency. At present, a variety of long-acting GLP-1 receptor agonists, such as Dulaglutide produced by Eli Lilly and Company and Semaglutide produced by Novo Nordisk, are available in the market and show significant advantages over traditional oral antidiabetic agents, short-acting GLP-1 receptor agonists or insulin products in the blood glucose control or patient compliance in clinical application. However, due to the limitation of mechanism, the long-acting GLP-1 receptor agonists perform corresponding effects mainly by promoting the secretion and release of insulin from pancreatic beta cells under the stimulation of glucose, the physiological function of the long-acting GLP-1 receptor agonists in glycometabolism mainly depends on insulin activity, and at present, there is no solid clinical evidence that these products can directly and definitely ameliorate insulin resistance, obesity, non-alcoholic fatty liver disease, hyperlipidemia or other diseases which diabetic patients universally suffer from through a mechanism of action independent of weight loss. This kind of products can play a certain role in weight loss by inhibiting the food intake of patients, but its weight loss effect mostly depends on the gastrointestinal adverse reactions caused by the products themselves. Although some positive treatment results have been achieved, the clinical discomfort of patients increases during the treatment.

Clinically, most patients with cardiovascular diseases and metabolic diseases also suffer from hyperlipidemia, obesity, hyperglycemia, fatty liver, atherosclerosis and other diseases. For example, epidemiological results show that about 60% of patients with type 2 diabetes also suffer from obesity, about 50% suffer from non-alcoholic liver diseases, and more than 70% have abnormal blood lipid levels; the risk of suffering from cardiovascular diseases in diabetic patients is 2 to 4 times that in non-diabetic patients, and cardiovascular events have become the first factor of all-cause death among diabetic patients; and cardiovascular events are the second cause of death among patients with non-alcoholic fatty liver disease. At present, multi-drug combination therapy is recommended in clinic to control the course of multiple diseases simultaneously. However, given the limitations of the therapeutic effects of approved drugs including GLP-1 Fc fusion proteins, no ideal monotherapy or combination therapy has been reported. Therefore, it is urgent to provide a more effective and comprehensive combined prevention and treatment solution for such patients. We creatively envisioned that the combination of FGF21 Fc fusion protein and GLP-1 Fc fusion protein may have great therapeutic potentials in the comprehensive management and treatment of patients with cardiovascular diseases and metabolic diseases and fully satisfy the demands of clinical efficacy.

### SUMMARY

An object of the present disclosure is to provide an FGF21 Fc fusion protein, a GLP-1 Fc fusion protein, and a combination therapeutic agent using the two proteins as an FGF21R/GLP-1R duel long-acting agonist for preventing and treating cardiovascular diseases and/or metabolic diseases, especially for providing a comprehensive management, prevention and treatment means for patients complicated with various cardiovascular diseases or metabolic diseases. The combination administration of the FGF21 Fc fusion protein and the GLP-1 Fc fusion protein provided by the present disclosure unexpectedly shows a remarkable synergistic effect in animal models of obesity, diabetes, hyperlipidemia, non-alcoholic fatty liver disease, atherosclerosis, diabetic cardiomyopathy and the like.

In one aspect, the present disclosure provides an FGF21 Fc fusion protein having an amino acid sequence:
(1) that is as shown in SEQ ID NO. 4; or
(2) that is substantially identical (e.g., a sequence having at least 80%, 85%, 90%, 92%, 95%, 97%, 98%, 99% or more identity or having one or more amino acid substitutions (e.g., conservative substitution), deletions and/or additions) to any of the above sequence.

In another aspect, the present disclosure provides a GLP-1 Fc fusion protein having an amino acid sequence:
(1) that is as shown in SEQ ID NO. 5, SEQ ID NO. 6 or SEQ ID NO. 7; or
(2) that is substantially identical (e.g., a sequence having at least 80%, 85%, 90%, 92%, 95%, 97%, 98%, 99% or more identity or having one or more amino acid substitutions (e.g., conservative substitution), deletions and/or additions) to any of the above sequences.

In another aspect, the present disclosure provides a combination therapeutic agent. The combination therapeutic agent is composed of a first pharmaceutical composition including a long-acting FGF21 Fc fusion protein and a second pharmaceutical composition including a long-acting GLP-1 Fc fusion protein, and wherein the long-acting FGF21 Fc fusion protein has an amino acid sequence selected from a sequence as shown in SEQ ID NO. 4 or a sequence substantially identical (e.g., a sequence having at least 80%, 85%, 90%, 92%, 95%, 97%, 98%, 99% or more identity or having one or more amino acid substitutions (e.g., conservative substitution)), deletions and/or additions to any of the above sequence, and the long-acting GLP-1 Fc fusion protein has an amino acid sequence selected from a sequence as shown in SEQ ID NO. 5, SEQ ID NO. 6 or SEQ ID NO. 7 or a sequence substantially identical (e.g., a sequence having at least 80%, 85%, 90%, 92%, 95%, 97%, 98%, 99% or more identity or having one or more amino acid substitutions (e.g., conservative substitution)), deletions and/or additions to any of the above sequences.

Further, the first pharmaceutical composition and the second pharmaceutical composition further include a pharmaceutically acceptable carrier and/or excipient and/or stabilizer.

Further, the FGF21 Fc fusion protein and the GLP-1 Fc fusion protein may be included in the pharmaceutical compositions in a prophylactically effective amount or therapeutically effective amount, wherein the prophylactic effective amount or the therapeutically effective amount is determined according to the therapeutic purpose or prophylactic purpose, for example, according to the condition of the patient in need of treatment, the required administration route, etc.

Further, the first pharmaceutical composition and the second pharmaceutical composition are formulated in a dosage form suitable for oral administration or administration by injection.

Further, the first pharmaceutical composition and the second pharmaceutical composition are separately formulated in dosage forms suitable for oral administration or administration by injection.

In another aspect, the present disclosure further provides use of the combination therapeutic agent in the preparation of a drug for the prevention or treatment of cardiovascular diseases and/or metabolic diseases.

Further, the cardiovascular diseases and/or metabolic diseases include, but are not limited to, obesity, hyperlipidemia, atherosclerosis, non-alcoholic fatty liver disease, diabetes, diabetic cardiomyopathy, coronary atherosclerotic heart disease, and other diseases associated with insulin resistance.

In another aspect, the present disclosure provides a method of using the combination therapeutic agent for the prevention and treatment of the above-mentioned diseases.

Further, the method includes administering a prophylactically or therapeutically effective amount of a first pharmaceutical composition and a second pharmaceutical composition in combination to a subject or a patient, wherein the prophylactically or therapeutically effective amount is determined according to the therapeutic purpose or prophylactic purpose, for example, according to the condition of the patient in need of treatment, the required administration route, etc.

Further, when the first pharmaceutical composition and the second pharmaceutical composition are administered in combination, the two pharmaceutical compositions are administered simultaneously and together, administered simultaneously but separately, or administered un-simultaneously, wherein when the pharmaceutical compositions are administered un-simultaneously, it means that the pharmaceutical compositions are successively administered or successively administered at intervals of a period of time.

Further, the first pharmaceutical composition and the second pharmaceutical composition may be administered in any manner known in the art, such as by injection such as intravenous (i.v.) or subcutaneous (s.c.) injection.

The present disclosure has the following advantages. In one aspect, from the perspective of the clinical actual treatment effect and the mechanism of action of drugs, the combination of FGF21R/GLP-1R duel agonists is applied to cope with the complexity of disease occurrence mechanism and satisfy the diversity of treatment requirements, and the advantages and feasibility of the combination of long-acting FGF21 Fc fusion protein and long-acting GLP-1 Fc fusion protein are fully clarified through scientific experimental research. The therapeutic method provided by the present disclosure optimizes the current clinical treatment means of cardiovascular diseases and metabolic diseases, and in particular, provides a more effective and comprehensive combination therapy for patients complicated with various cardiovascular or metabolic diseases. In another aspect, the active ingredients of the combination therapeutic agent selected by the preset disclosure are all long-acting protein drugs, which all can realize the administration frequency once a week in clinic, and the patient acceptance of the combination therapeutic agent is much higher than that of other insulin or GLP-1 analog products.

### Detailed description

The term "FGF21" refers to natural human FGF21 as well as analogues and derivatives thereof that maintain the activity of FGF21.

The sequence of the natural human FGF21 protein can be obtained from UNIPROT database with the accession number of Q9NSA1. The precursor protein consists of 209 amino acids, including a signal peptide (amino acids 1-28) and a mature protein (amino acids 29-209).

An isoform or allelic form of the natural human FGF21 having Pro instead of Leu in the mature protein (at position 174 of SEQ ID NO. 1 provided by the present disclosure) can be known, in particular, from US2001012628A1. There is another natural human FGF21 isoform where Gly is substituted with Ser (at position 141 of SEQ ID NO. 1 provided by the present disclosure).

Another isoform having a short signal peptide (Leu at position 23 of SEQ ID NO. 1 provided by the present disclosure is missing) can be known from WO2003/011213 (see SEQ ID NO. 2 disclosed by WO2003/011213 which has a signal peptide of 27 amino acid residues).

In the present disclosure, the natural human FGF21 includes a sequence of the mature protein part (amino acids 29-209) obtained after the leader peptide is removed from SEQ ID NO. 1 and L174P or G141S substitution isoform, and in addition, the natural human FGF21 further includes the full-length sequence of the precursor protein obtained by adding before the preceding sequences the above-mentioned signal peptide of 27 or 28 amino acids.

The term "GLP-1" refers to human GLP-1 (7-37) (amino acids 1-31 of SEQ ID NO. 2), exendin-4 (7-45) (amino acids 1-39 of SEQ ID NO. 3), and analogues and derivatives thereof maintaining GLP-1 activity.

The terms "FGF21 analogue" and "GLP-1 analogue" refer to polypeptides that are or can be, respectively, deduced or derived from respective FGF21, GLP-1 and Exendin-4 sequences of SEQ ID NOs. 1, 2 and 3 by modification of the amino acid sequences thereof. Such modification may include one or more amino acid substitutions, deletions and/or additions. For example, amino acids may be added and/or deleted at the C-terminus, N-terminus or inside the amino acid sequence. Preferably, amino acids are added and/or deleted at the C-terminus and/or the N-terminus, more preferably at the N-terminus. Amino acid sequences with the deletion of C-terminus or N-terminus-amino acids may also be referred to as a truncated sequence, as is known in the art.

The pharmaceutical compositions including the FGF21 Fc fusion protein and the GLP-1 fusion protein of the present disclosure may further include a pharmaceutically acceptable carrier. For injection, the carrier may be, for example, water or normal saline. Other pharmaceutically acceptable substances such as diluents and appropriate buffers may also be used. Other pharmaceutically acceptable substances, such as emulsifiers, suspending agents, solvents, fillers, swelling agents, adjuvants, preservatives, antioxidants, colorants and/or flavoring agents, may also be used if needed. The FGF21 Fc fusion protein and the GLP-1 Fc fusion protein may be used in the form of a purified polypeptide or formulated with a suitable pharmaceutically acceptable excipient, as is known in the art. The pharmaceutical compositions may be administered in any manner known in the art, such as by injection such as intravenous (i.v.) or subcutaneous (s.c.) injection.

The FGF21 Fc fusion protein and the GLP-1 Fc fusion protein may be included in the pharmaceutical compositions in a prophylactically effective amount or therapeutically effective amount. The prophylactic effective amount or the therapeutically effective amount is determined according to the prophylactic purpose or therapeutic purpose, for example, according to the condition of the patient in need of treatment, the required administration route, etc.

The term "insulin resistance" refers to a state in which normal doses of insulin produce a biological effect lower than the normal biological effect, which slows down the uptake of glucose by fat and skeletal muscle but increases the release of glucose from the liver and free fatty acids from the adipose tissue. The first response of insulin resistance is the compensatory production and secretion of insulin to compensate for the decreased sensitivity of the body, causing hyperinsulinemia. Therefore, insulin resistance is characterized by high insulin levels and decreased responsiveness of tissues to remove glucose from the circulation. Insulin resistance is the main cause of a series of metabolic changes, including compensatory hyperinsulinemia, dyslipidemia, pancreatic beta cell compensatory hypofunction and hyperglycemia.

Diseases related to insulin resistance are specifically selected from insulin resistance syndrome (IRS), type 2 diabetes, impaired glucose tolerance, metabolic syndrome, hyperglycemia, hyperinsulinemia, arteriosclerosis, hypercholesterolemia, hypertriglyceridemia, hyperlipidemia, dyslipidemia, obesity, central obesity, polycystic ovary syndrome, excessive coagulation, hypertension and microalbuminuria.

The term "diabetes" is an endocrine and metabolic disease caused by absolute or relative deficiency of insulin secretion. The pathogenesis of type 2 diabetes includes the progressive development of insulin resistance in the liver and peripheral tissues, accompanied by insulin secretion defect of pancreatic beta cells, resulting in obvious hyperglycemia (abnormally high level of glucose in the blood).

The term "diabetes complications" refers to dysfunction in other parts of the body induced by chronic hyperglycemia, such as diabetic nephropathy, diabetic neuropathy, diabetic foot (foot ulcer and low blood circulation) and eye diseases (retinopathy). Diabetes also increases the risk of heart disease and bone and joint diseases. Other long-term complications of diabetes include skin disorders, digestive disorders, sexual dysfunction and tooth and gum disorders.

The term "diabetic cardiomyopathy" refers to a disorder of the heart muscle in people with diabetes, which cannot be explained by hypertensive heart disease, coronary atherosclerotic heart disease or other heart diseases. This disease induces extensive focal myocardial necrosis on the basis of metabolic disorders and microangiopathy, leads to subclinical cardiac dysfunction, and finally progresses to heart failure, arrhythmia, cardiogenic shock, and even sudden death of critical patients.

The term "coronary atherosclerotic heart disease" is a heart disease caused by atherosclerotic lesions in the coronary arteries vessel which leads to stenosis or obstruction of vascular lumens, resulting in myocardial ischemia, hypoxia or necrosis.

The term "dyslipidemia" is a disorder of lipoprotein metabolism, including lipoprotein overproduction or defect. Dyslipidemia can be characterized by the increase in total cholesterol, low density lipoprotein (LDL) cholesterol and triglyceride, and the decrease in high density lipoprotein (HDL) cholesterol in blood.

The term "non-alcoholic fatty liver disease" or "NAFLD" refers to all diseases of the liver caused by steatosis rather than excessive alcohol use. NAFLDs include, but are not limited to, simple non-alcoholic fatty liver (NAFL), non-alcoholic steatohepatitis (NASH), NAFL with liver fibrosis, NAFL with liver cirrhosis, NASH with liver fibrosis, NASH with liver cirrhosis, and fatty liver diseases caused by hepatitis, obesity, diabetes, insulin resistance, hypertriglyceridemia, lipoproteinemia, glycogen storage diseases, Weber-Christian disease, Wolman's disease, pregnancy or lipodystrophy.

The term "non-alcoholic steatohepatitis (NASH)" is a liver disease unrelated to alcohol consumption and characterized by hepatic steatosis accompanied by intralobular inflammation and fibrosis.

The term "atherosclerosis (AS)" is a systemic disease related to lipid metabolism disorder, which is characterized in that lipids in blood enter the wall of the arterial and deposit in the intima to form atheromatous plaques, resulting in thickening and hardening of arteries. AS mainly involves medium and large arteries, and its basic lesions are lipidosis, focal fibrosis and the formation of atheromatous plaques in arterial intima, which leads to hardening of walls and narrowing of lumens and induces a series of secondary diseases, especially in heart, brain, kidney and other organs, resulting in ischemic changes.

The term "pharmaceutically acceptable carrier and/or excipient and/or stabilizer" refers to a carrier and/or excipient and/or stabilizer that are pharmacologically and/or physiologically compatible with a subject and an active ingredient and are non-toxic to cells or mammals exposed thereto in doses and concentrations used. The pharmaceutically acceptable carrier and/or excipient and/or stabilizer include, but are not limited to, a PH adjusting agent, a surfactant, an adjuvant, an ionic strength enhancer, a diluent, a reagent for maintaining osmotic pressure, a reagent for delaying absorption, and a preservative. For example, the pH adjusting agent includes, but is not limited to, a phosphate buffer. The surfactant includes, but is not limited to, cationic, anionic or non-ionic surfactants, such as Tween-80. The ionic strength enhancer includes, but is not limited to, sodium chloride. The preservative includes, but is not limited to, various antibacterial reagents and antifungal reagents, such as parabens, chlorobutanol, phenol, and sorbic acid. The reagent for maintaining osmotic pressure includes, but is not limited to, sugar, NaCl and analogues thereof. The reagent for delaying absorption includes, but is not limited to, a monostearate and gelatin. The diluent includes, but is not limited to, water, aqueous buffers (such as buffered saline), alcohols and polyols (such as glycerol). The preservative includes, but is not limited to, various antibacterial reagents and antifungal reagents such as thimerosal, 2-phenoxyethanol, parabens, chlorobutanol, phenol and sorbic acid. The stabilizer has the meaning generally understood by those skilled in the art, can stabilize the desired activity of active ingredients in a drug, and includes, but is not limited to, sodium glutamate, gelatin, SPGA, sugars (such as sorbitol, mannitol, starch, sucrose, lactose, dextran and glucose), amino acids (such as glutamate and glycine), proteins (such as dried whey, albumin or casein), or degradation products thereof (such as lactalbumin hydrolysate).

The terms "patient", "subject", "individual" and "object" refer to any human or non-human animal, in particular human, that receives prophylactic or therapeutic treatment. For example, the combination therapeutic agent and the method described herein may be used for treating subjects with diabetes, NASA, NAFLD, or atherosclerotic diseases. The term "non-human animal" includes all vertebrates, for example, mammals and non-mammals, such as non-human primates, sheep, dogs, cattle, chickens, amphibians and reptiles.

The term "effective amount" refers to an amount sufficiently or at least partially to obtain the expected effect. For example, the effective amount for the prevention of diseases (such as tumor or infection) refers to an amount sufficient to prevent, arrest, or delay the onset of a disease (such as tumor or infection) when used alone or used in combination with one or more therapeutic agents; the effective amount for the treatment of diseases refers to an amount sufficient to cure or at least partially arrest a disease and complications thereof in a patient who has already suffered from the disease when used alone or used in combination with one or more therapeutic agents. The determination of such an effective amount is well within the abilities of those skilled in the art. For example, the amount effective for therapeutic use depends on the severity of the disease to be treated, the overall state of the immune system of the patient, the general condition of the patient such as age, weight and sex, the mode of administration of the drug, and other concurrent treatment. The terms "effectiveness" and "efficacy" with respect to treatment include both pharmacological efficacy and physiological safety. The pharmacological effectiveness refers to the ability of a drug to promote regression of a disease or symptom in a patient. The physiological safety refers to the level of toxicity or other adverse physiological effects (adverse effects) at the cellular, organ and/or organism level due to drug administration.

The "treatment" or "therapy" for a subject refers to any type of intervention or treatment on the subject or the administration of the combination therapeutic agent of the present disclosure to the subject for the purpose of reversing, alleviating, improving, inhibiting, delaying or preventing the occurrence, progression, development, severity or recurrence of symptoms, complications, conditions or biochemical indicators related to a disease.

### BRIEF DESCRIPTION OF DRAWINGS

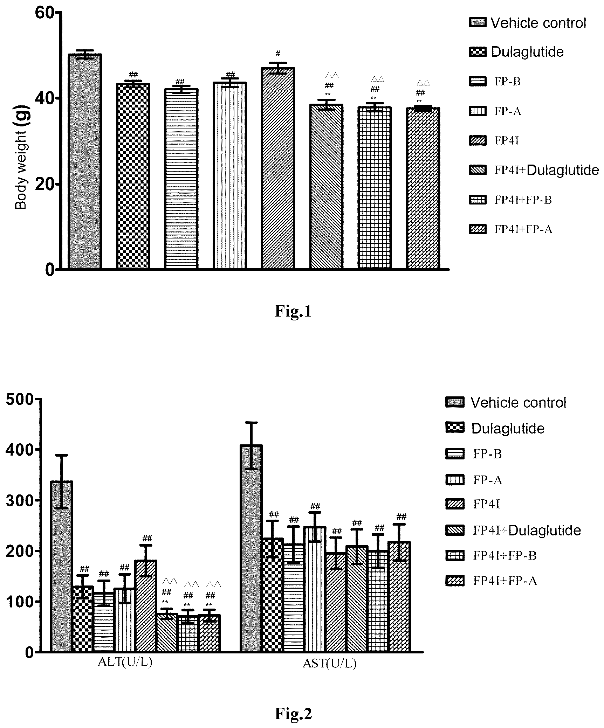

FIG. 1 shows effects of the combination of FP4I respectively with GLP-1 Fc fusion protein Dulaglutide, FP-B and FP-A on body weight of obese mice (means ± SEM), where the annotations of statistical difference markers are as follows: compared with the vehicle control group, ^{##}*P* < *0.01;* compared with the GLP-1 Fc fusion protein alone group, ^{∗∗}*P* < *0.01*; and compared with the FP4I alone group, *^{ΔΔ}P* < *0.01.*
FIG. 2 shows effects of the combination FP4I respectively with GLP-1 Fc fusion protein Dulaglutide, FP-B and FP-A on the serum liver function of obese mice (means ± SEM), where the annotations of statistical difference markers are as follows: compared with the vehicle control group, *^{##}P* < *0.01*; compared with the GLP-1 Fc fusion protein alone group, ^{∗∗}*P* < *0.01*; and compared with the FP4I alone group, *^{ΔΔ}P* < *0.01.*

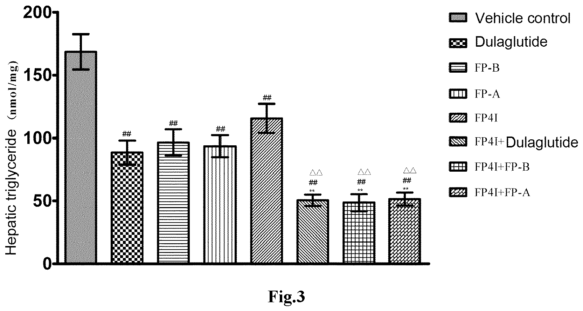

FIG. 3 shows effects of the combination of FP4I respectively with GLP-1 Fc fusion protein Dulaglutide, FP-B and FP-A on the hepatic triglyceride content of obese mice (means ± SEM), where the annotations of statistical difference markers are as follows: compared with the vehicle control group, ^{##}*P* < *0.01*; compared with the GLP-1 Fc fusion protein alone group, ^{∗∗}*P* < *0.01;* and compared with the FP4I alone group, *^{ΔΔ}P* < *0.01.*

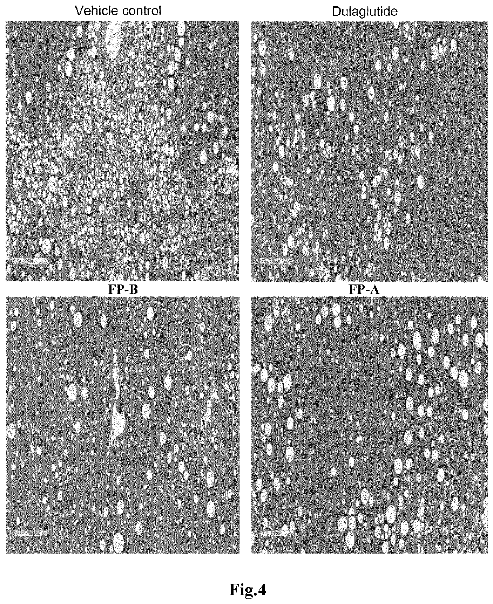

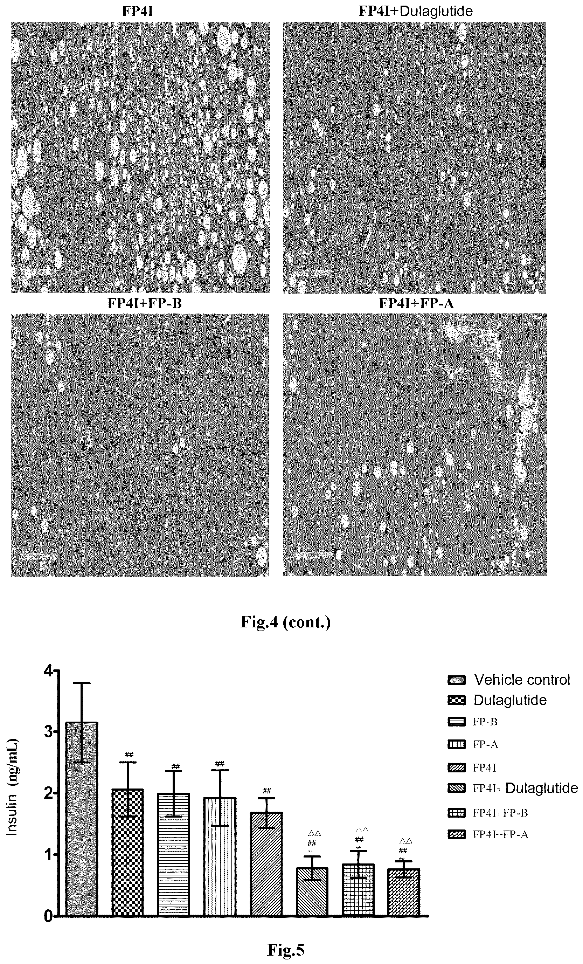

FIG. 4 shows pathology images of the liver tissue of obese mice to which FP4I was used respectively in combination with GLP-1 Fc fusion protein Dulaglutide, FP-B and FP-A.
FIG. 5 shows effects of the combination of FP4I respectively with GLP-1 Fc fusion protein Dulaglutide, FP-B and FP-A on the serum insulin level of obese mice (means ± SEM), where the annotations of statistical difference markers are as follows: compared with the vehicle control group, ^{##}*P* < *0.01;* compared with the GLP-1 Fc fusion protein alone group, ^{∗∗}*P* < *0.01*; and compared with the FP4I alone group, *^{ΔΔ}P* < *0.01.*

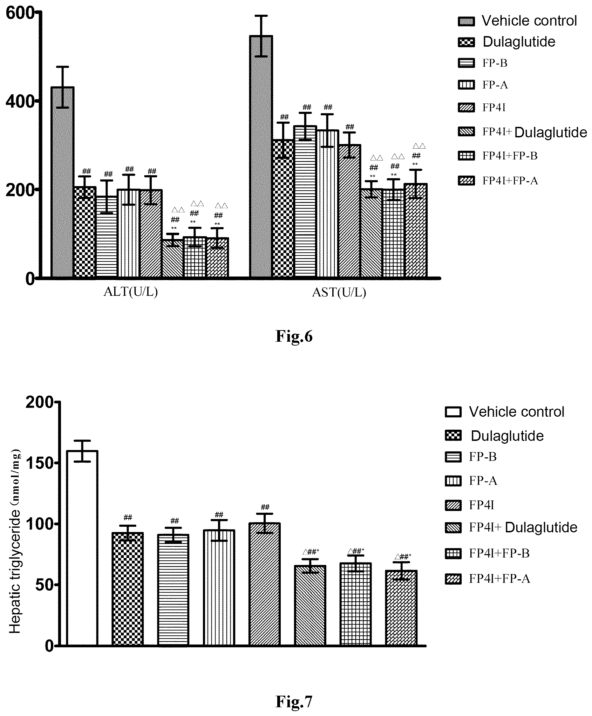

FIG. 6 shows effects of the combination of FP4I respectively with GLP-1 Fc fusion protein Dulaglutide, FP-B and FP-A on the serum liver function of NASH mice (means ± SEM), where the annotations of statistical difference markers are as follows: compared with the vehicle control group, ^{##}*P* < *0.01;* compared with the GLP-1 Fc fusion protein alone group, ^{∗∗}*P* < *0.01*; and compared with the FP4I alone group, *^{ΔΔ}P* < *0.01.*
FIG. 7 shows effects of the combination of FP4I respectively with GLP-1 Fc fusion protein Dulaglutide, FP-B and FP-A on the hepatic triglyceride content of NASH mice (means ± SEM), where the annotations of statistical difference markers are as follows: compared with the vehicle control group, ^{##}*P* < *0.01*; compared with the GLP-1 Fc fusion protein alone group, ^{∗}*P* < *0.05*; and compared with the FP4I alone group, *^{Δ}P* < *0.05.*

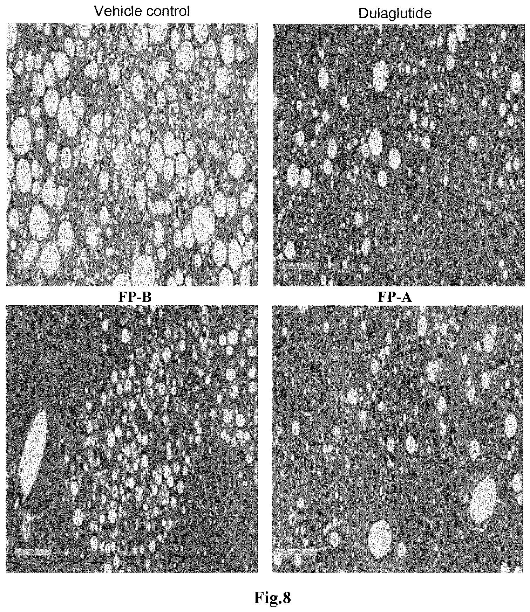

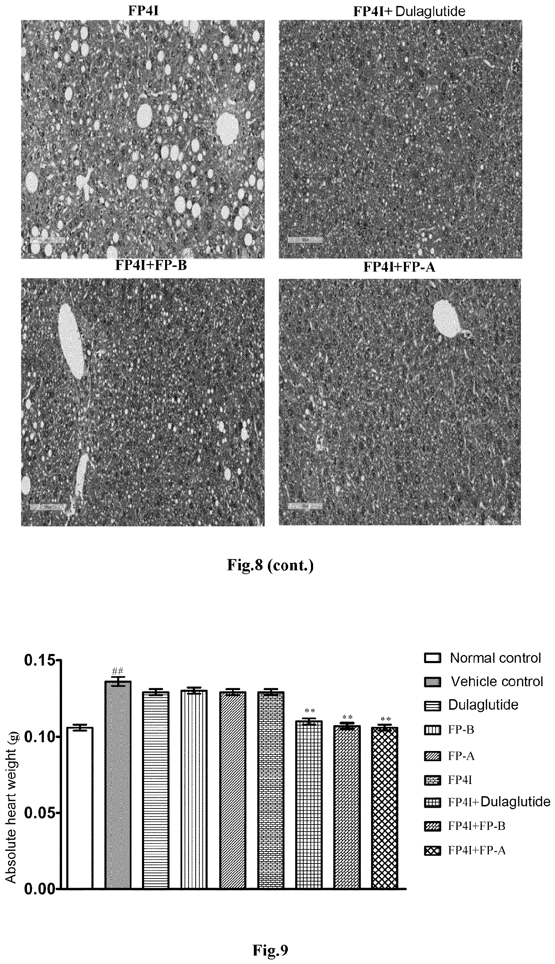

FIG. 8 shows pathology images of the liver tissue of NASH mice to which FP4I was used respectively in combination with GLP-1 Fc fusion protein Dulaglutide, FP-B and FP-A.
FIG. 9 shows effects of the combination of FP4I respectively with GLP-1 Fc fusion protein Dulaglutide, FP-B and FP-A on the absolute heart weight of db/db mice (means ± SEM), where the annotations of statistical difference markers are as follows: compared with the normal control group, ^{##}*P* < *0.01*; and compared with the vehicle control group, ^{∗∗}*P* < *0.01.*

### EXAMPLE

Through the combination of a long-acting FGF21-Fc fusion protein (named FP4I, see Chinese patent CN107995914A) respectively with a long-acting GLP-1 Fc fusion protein Dulaglutide (trade name: Trulicity), Ex(1-39)-L3-CTP¹-vFCγ₂₋₃ (named FP-A, see Chinese patent CN106117370B) or Ex-(1-45)-L-vFc (named FP-B, see Chinese patent CN106279430B), using animal models of obesity, hyperlipidemia, non-alcoholic fatty liver disease or diabetes as research objects, the present disclosure fully clarifies the therapeutic advantages of the combination of the two products and provides a more effective and comprehensive solution for the comprehensive management, prevention and treatment of cardiovascular diseases and metabolic diseases.

The present disclosure is further described below in conjunction with specific examples. It is to be understood that the examples described below are merely intended to explain and not to limit the scope of the present disclosure.

### Example 1 Therapeutic effect of the combination on obesity, lipid metabolism disorder and fatty liver induced by a high-fat diet in mice

### 1.1 Sources of Drugs

Long-acting FGF21-Fc fusion protein (named FP4I, whose preparation method can be found in CN107995914A and the amino acid sequence of which in the present disclosure is shown in SEQ ID NO. 4), Dulaglutide (produced by Lily Company, USA, the amino acid sequence of which in the present disclosure is shown in SEQ ID NO. 5), Ex(1-39)-L3-CTP¹-vFCγ₂₋₃ (named FP-A, whose preparation method can be found in Chinese patent CN106117370B and amino acid sequence of which in the present disclosure is shown in SEQ ID NO. 6), Ex-(1-45)-L-vFc (named FP-B, whose preparation method can be found in Chinese patent CN106279430B and amino acid sequence of which in the present disclosure is shown in SEQ ID NO. 7).

### 1.1 Grouping, drug administration and samples collection

Eight-week-old male C57BL/6J mice were purchased from Shanghai SLAC Laboratory Animal Co., Ltd. The mice were fed in an environment of 22 °C to 24 °C with relative humidity of 45% to 65% and lighting time of 12 h/day. The mice were, after fed a D12492 diet (60% fat calorie, produced by Research Diets, USA) for 20 weeks, randomly divided into a vehicle control group, an FP4I-5mg/kg group (FP4I group), a Dulaglutide-1.5mg/kg group (Dulaglutide group), FP-B-1.5mg/kg group (FP-B group), FP-A-1.5mg/kg group (FP-A group), FP4I-5mg+Dulaglutide-1.5mg/kg group (FP4I+Dulaglutide group), FP4I-5mg/kg+FP-B-1.5mg/kg group (FP4I+FP-B group) and FP4I-5mg+FP-A-1.5mg/kg group (FP4I+FP-A group), eight mice per group. The mice in the vehicle control group and the monotherapy groups were subcutaneously injected with corresponding protein solutions or buffers to the nape of the neck. The mice in the combination groups were subcutaneously injected with FP4I at the nape and subcutaneously injected with Dulaglutide, FP-A or FP-B at the abdomen. The mice were administered once every six days, a total of four times. After the last administration, the mice in each group were fasted for 16 hours, and the whole blood was collected from the orbit and centrifuged at 2000 g for 15 minutes for separation to obtain the serum samples. The liver tissue was separated and weighed, and the left lateral lobe of liver was frozen in liquid nitrogen and then transferred to the refrigerator at -80 °C for preservation while the right lobe of liver was preserved in 10% formalin solution.

### 1.2 Experimental method

ALT, AST, TG, TC, LDL-c and HDL-c content in serum of mice were detected by an automatic biochemical analyzer (ERBA XL-200 automatic biochemical analyzer, produced by Erba Tech, Germany) and the supporting kit (produced by Ningbo Medicalsystem Biotechnology Co., Ltd.). The fasting serum insulin content of mice was detected by ELISA method (using Mouse Ultrasensitive Insulin ELISA kit, produced by ALPCO, USA). About 55 mg of liver tissue was collected from the left lateral lobe of liver of mice, and the content of triglyceride per unit liver mass was detected by Floch method. The right lobe of liver of mice was taken, stained with HE, and subjected to histopathological examination.

The data were expressed in the form of mean ± standard error (means ± SEM), and analyzed by SPSS 18.0 statistical software. In a case of normal distribution, mean comparison in groups was conducted with single factor variance analysis, and LSD test was conducted when there was homogeneity in the variances and Dunnet T3 test was conducted when there was heterogeneity in the variances, while in a case of non-normal distribution, the non-parametric test was conducted, and *P* < *0.05* indicated that there was statistically significant difference.

### 1.3 Experimental result

Results are shown in FIG. 1. Compared with the vehicle control group, the body weight of mice in the Dulaglutide group, FP-A group, FP-B group and FP4I group was significantly decreased. Compared with the groups in which mice were administrated with FGF21 Fc fusion protein alone or GLP-1 Fc fusion protein alone, the body weight of mice in the combination groups was further significantly reduced. Results are shown in FIG. 2 and FIG. 3. The improvement degree of serum liver function of mice in the combination groups was significantly better than that in the monotherapy groups, and the hepatic triglyceride content was significantly lower than that in the monotherapy groups.

Histopathologic results show that the liver of the mice in the vehicle control group showed a mix of macrovesicular steatosis and microvesicular steatosis, and the lipid droplets fused into flakes. The administration of Dulaglutide, FP-B, FP-A or FP4I alone had an effect of ameliorating hepatic steatosis in mice. Compared with the administration of FGF21 fusion protein or GLP-1 Fc fusion protein alone, the degree of hepatic steatosis in mice in the combination groups was alleviated to a greater extent, as shown in FIG. 4.

Results are shown in Table 1 and Table 2. The combination of FGF21 fusion protein and GLP-1 Fc fusion protein had better therapeutic effect on hyperlipidemia in obese mice than the monotherapy group.

**Table 1. Effects of the combination of FGF21 Fc fusion protein and long-acting GLP-1 Fc fusion protein on serum TG and TC contents in obese mice**

| Group | TG (mmol/L) | TC (mmol/L) |
|---|---|---|
| Vehicle control | 1.24 ± 0.08 | 6.95 ± 0.4 |
| Dulaglutide | 1.08 ± 0.06 | 4.82 ± 0.03^{##} |
| FP-B | 1.07 ± 0.04 | 4.93 ± 0.04^{##} |
| FP-A | 1.11 ± 0.04 | 4.88 ± 0.04^{##} |
| FP4I | 1.09 ± 0.06 | 4.74 ± 0.04^{##} |
| FP4I+Dulaglutide | 0.84 ± 0.07^{##^{∗}△} | 4.01 ± 0.03^{##^{∗}△} |
| FP4I+FP-B | 0.82 ± 0.06^{##^{∗}△} | 3.94 ± 0.02^{##^{∗}△} |
| FP4I+FP-A | 0.87 ± 0.04^{##^{∗}△} | 3.98 ± 0.03^{##^{∗}△} |

Remarks: Compared with the vehicle control group, ^{#}*P* < *0.05*, and ^{##}*P* < *0.01*; compared with the GLP-1 Fc fusion protein alone group, ^{∗}*P* < *0.05*, and ^{∗∗}*P* < *0.01*; compared with the FP4I alone group, ^{△}*P* < *0.05*, and ^{△△}*P* < *0.01*; and the statistical annotations of other tables are the same as those in Table 1.

**Table 2. Effects of the combination of FP4I and GLP-1 Fc fusion protein on serum HDL-c and LDL-c contents in obese mice**

| Group | HDL-c (mmol/L) | LDL-c (mmol/L) |
|---|---|---|
| Vehicle control | 2.19 ± 0.07 | 1.71 ± 0.15 |
| Dulaglutide | 2.02 ± 0.06 | 0.88 ± 0.09^{##} |
| FP-B | 1.98 ± 0.06 | 0.91 ± 0.08^{##} |
| FP-A | 2.04 ± 0.05 | 0.86 ± 0.09^{##} |
| FP4I | 1.96 ± 0.07 | 0.93 ± 0.1^{##} |
| FP4I+Dulaglutide | 1.72 ± 0.05^{##^{∗}△} | 0.61 ± 0.07^{##^{∗}△} |
| FP4I+FP-B | 1.71 ± 0.06^{##^{∗}△} | 0.57 ± 0.08^{##^{∗}△} |
| FP4I+FP-A | 1.69 ± 0.07^{##^{∗}△} | 0.58 ± 0.07^{##^{∗}△} |

As shown in FIG. 5, when FP4I, Dulaglutide, FP-B and FP-A was used alone, the symptoms of hyperinsulinemia in obese mice were effectively ameliorated. When FP4I was used in combination with GLP-1 Fc fusion protein, the effect of FP4I on the amelioration of insulin resistance was significantly enhanced.

The research content of this example shows that, with obese mice with typical metabolic syndrome as the research object, the combination of long-acting FGF21 fusion protein and long-acting GLP-1 Fc fusion protein had more excellent therapeutic effects on weight loss, reversion of hepatic steatosis and liver injuries induced by fatty liver, relief and treatment of vascular degenerative diseases induced by dyslipidemia, insulin resistance and low density lipoprotein elevation than monotherapy.

### Example 2 Therapeutic effect of the combination on non-alcoholic steatohepatitis induced by a high-fructose, high-fat and high-cholesterol diet in mice

### 2.1 Grouping, drug administration and samples collection

Eight-week-old male C57BL/6J mice were purchased from Beijing HFK Bioscience Co., Ltd. The mice were fed in an environment of 22 °C to 24 °C with relative humidity of 45% to 65% and lighting time of 12 h/day. The mice were, after fed a D09100301 diet (40% fat calorie, 40% fructose calorie, 2% cholesterol mass, produced by Research Diets, USA) for 30 weeks, fasted for 12 hours, and about 120 µl of whole blood was collected from the venous plexus of the inner canthus and subjected to serum separation to detect the ALT level. According to the body weight and serum ALT level, the mice were randomly divided into a vehicle control group, an FP4I-5mg/kg group (FP4I group), a Dulaglutide-1.5mg/kg group (Dulaglutide group), FP-B-1.5mg/kg group (FP-B group), FP-A-1.5mg/kg group (FP-A group), FP4I-5mg+Dulaglutide-1.5mg/kg group (FP4I+Dulaglutide group), FP4I-5mg/kg+FP-B-1.5mg/kg group (FP4I+FP-B group) and FP4I-5mg+FP-A-1.5mg/kg group (FP4I+FP-A group), eight mice per group. The mice in the vehicle control group and the monotherapy groups were subcutaneously injected with corresponding protein solutions or buffers at the nape. The mice in the combination groups were subcutaneously injected with FP4I at the nape and subcutaneously injected with Dulaglutide, FP-A or FP-B at the abdomen. The mice were administered once every six days, a total of eight times. After the last administration, the mice in each group were fasted for 16 hours, and the whole blood was collected from the venous plexus of the inner canthus and centrifuged at 2000 g for 15 minutes for separation to obtain the serum samples. The liver tissue was separated and weighed, and the left lateral lobe of liver was frozen in liquid nitrogen and then transferred to the refrigerator at -80 °C for preservation while the right lobe of liver was preserved in 10% formalin solution.

### 2.2 Experimental method

The therapeutic effect was evaluated by non-alcoholic steatohepatitis score system (NAS score) in accordance with pathology working group guidelines from National Institutes of Health, which was specifically as follows: hepatocyte steatosis: 0 (< 5%), 1 (5% to 33%), 2 (34% to 66%) and 3 (> 66%); intralobular inflammation under 20X: 0 (none), 1 (< 2), 2 (2 to 4), 3 (< 4); and ballooning degeneration of hepatocytes: 0 (none), 1 (rare) and 2 (common). Serum biochemical assay, hepatic triglyceride content test, histopathological examination and statistical analysis were performed in the same manners as in Example 1.

### 2.3 Experimental result

As shown in FIG. 6 and FIG. 7, on the basis of the treatment of monotherapy, the combination can further improve the liver function of mice with non-alcoholic steatohepatitis and reduce the hepatic triglyceride content.

Histopathologic results show that the liver of the mice in the vehicle control group showed severe steatosis, inflammatory cell infiltration and occasional ballooning degeneration of hepatocytes. Under the dosage and administration period designed in this example, the administration of FGF21 Fc fusion protein FP4I or GLP-1 Fc fusion protein Dulaglutide, FP-A or FP-B alone can alleviate liver lesions in mice with non-alcoholic steatohepatitis to a certain extent. Compared with the monotherapy groups, the liver pathological morphology of mice in the combination groups was significantly ameliorated, and NAS score was significantly decreased. The results are shown in FIG. 8 and Table 3.

The research content of this example shows that the combination of FGF21 Fc fusion protein and GLP-1 Fc fusion protein had a more positive therapeutic effect on non-alcoholic steatohepatitis, which was remarkably better than that of the monotherapy groups.

**Table 3. NAS score results**

| Group | NAS score |
|---|---|
| Vehicle control | 5.25 ± 0.25 |
| Dulaglutide | 3.875 ± 0.52^{##} |
| FP-B | 4 ± 0.46^{##} |
| FP-A | 3.75 ± 0.37^{##} |
| FP4I | 3.63 ± 0.42^{##} |
| FP4I+Dulaglutide | 1.75 ± 0.25^{##^{∗∗}△△} |
| FP4I+FP-B | 1.75 ± 0.31^{##^{∗∗}△△} |
| FP4I+FP-A | 1.88 ± 0.4^{##^{∗∗}△△} |

### Example 3. Experimental study on hypoglycemic and cardiac protective effects of the combination on db/db mice

### 3.1 Grouping, drug administration and samples collection

Six-week-old male db/db mice were purchased from Jiangsu GemPharmatech Co., Ltd. The mice were fed in an environment of 22 °C to 24 °C with relative humidity of 45% to 65% and lighting time of 12 h/day. The mice were fed a D12450B diet to 14 weeks old, and about 20 µl of whole blood was collected from the venous plexus of the inner canthus to detect the content of glycosylated hemoglobin. According to the body weight and glycosylated hemoglobin level, the db/db mice were randomly divided into a vehicle control group, an FP4I-5mg/kg group (FP4I group), a Dulaglutide-1.5mg/kg group (Dulaglutide group), FP-B-1.5mg/kg group (FP-B group), FP-A-1.5mg/kg group (FP-A group), FP4I-5mg+Dulaglutide-1.5mg/kg group (FP4I+Dulaglutide group), FP4I-5mg/kg+FP-B-1.5mg/kg group (FP4I+FP-B group) and FP4I-5mg+FP-A-1.5mg/kg group (FP4I+FP-A group), and db/m mice of the same weeks of age were used as the normal control group, eight mice per group. The mice in the control groups and the monotherapy groups were subcutaneously injected with corresponding protein solutions or buffers at the nape. The mice in the combination groups were subcutaneously injected with FP4I on the nape and subcutaneously injected with Dulaglutide, FP-A or FP-B at the abdomen. The mice were administered once every three days, a total of 12 times. After the last administration, the mice in each group were fasted overnight, and the whole blood was collected from the venous plexus of the inner canthus to detect the content of glycosylated hemoglobin. After the blood collection, the mice were sacrificed by cervical dislocation, heart tissue of the mice was separated, and the absolute heart weight was weighed.

### 3.2 Experimental method

The content of glycosylated hemoglobin in mice was detected by microparticle chromatography with NycoCard Reader II special protein gold standard detector (produced by Alere Technologies AS, Norway). The statistical analysis was performed in the same manners as in Example 1.

### 3.3 Experimental results

Results are shown in Table 4. The administration of FGF21 Fc fusion protein or GLP-1 Fc fusion protein alone showed good blood glucose control effect on db/db mice, but the blood glucose was still higher than the blood glucose level of normal mice. The content of glycosylated hemoglobin in db/db mice subjected to the combination therapy was significantly lower than that in the monotherapy groups, and the glycosylated hemoglobin level basically recovered to the blood glucose level of normal mice.

db/db mice were spontaneous diabetic mice whose symptoms were very similar to clinical symptoms, so these mice can accurately reflect the therapeutic effect of hypoglycemic drugs. The study of this example shows that the combination can make the blood glucose of db/db mice return to normal, which was better to the monotherapy groups.

Diabetic cardiomyopathy is one of the main complications of diabetes, and its main pathological feature is cardiac hypertrophy. Results are shown in FIG. 9. The absolute heart weight of 20-week-old db/db mice was significantly higher than that of normal control mice, showing the characteristics of diabetic cardiomyopathy. The administration of FGF21 Fc fusion protein or GLP-1 Fc fusion protein alone had no significant effect on the absolute heart weight of db/db mice. After the combination therapy, the absolute heart weight of db/db mice was significantly decreased. The study of this example shows that the combination can prevent the occurrence and development of diabetic cardiomyopathy and treat diabetic cardiomyopathy.

**Table 4. Glycosylated hemoglobin content**

| Group | Glycated hemoglobin (%) |
|---|---|
| Normal control | < 4 |
| Vehicle control | 10.12 ± 0.34 |
| Dulaglutide | 7.78 ± 0.24^{#} |
| FP-B | 7. 81 ± 0.33^{#} |
| FP-A | 7.95 ± 0.17^{#} |
| FP4I | 8.45 ± 0.46^{#} |
| FP4I+Dulaglutide | 4.81 ± 0.26^{##^{∗∗}△△} |
| FP4I+FP-B | 4.26 ± 0.33^{##^{∗∗}△△} |
| FP4I+FP-A | 4.54 ± 0.23^{##^{∗∗}△△} |

All the publications mentioned in the present disclosure are incorporated herein by reference as if each publication is separately incorporated herein by reference. In addition, it should be understood that those skilled in the art, who have read the present disclosure, can make various changes or modifications to the present disclosure, and these equivalent forms fall within the scope of the appended claims.

## Claims

1. An FGF21 Fc fusion protein, having an amino acid sequence:
(1) that is as shown in SEQ ID NO.4; or
(2) that is substantially identical (e.g., a sequence having at least 80%, 85%, 90%, 92%, 95%, 97%, 98%, 99% or more identity or having one or more amino acid substitutions (e.g., conservative substitution), deletions and/or additions) to any of the above sequence.

2. A GLP-1 Fc fusion protein, having an amino acid sequence:
(1) that is as shown in SEQ ID NO. 5, SEQ ID NO. 6 or SEQ ID NO. 7; or
(2) that is substantially identical (e.g., a sequence having at least 80%, 85%, 90%, 92%, 95%, 97%, 98%, 99% or more identity or having one or more amino acid substitutions (e.g., conservative substitution), deletions and/or additions) to any of the above sequences.

3. A combination therapeutic agent, consisting of a first pharmaceutical composition comprising the FGF21 Fc fusion protein of claim 1 and a second pharmaceutical composition comprising the GLP-1 Fc fusion protein of claim 2.

4. The combination therapeutic agent of claim 3, wherein the FGF21 Fc fusion protein and the GLP-1 Fc fusion protein are comprised in the first pharmaceutical composition and the second pharmaceutical composition in a prophylactically effective amount or therapeutically effective amount.

5. The combination therapeutic agent of claim 3, wherein the first pharmaceutical composition further comprises a pharmaceutically acceptable carrier and/or excipient and/or stabilizer.

6. The combination therapeutic agent of claim 3, wherein the second pharmaceutical composition further comprises a pharmaceutically acceptable carrier and/or excipient and/or stabilizer.

7. The combination therapeutic agent of any one of claims 3 to 6, wherein the first pharmaceutical composition and the second pharmaceutical composition are formulated in a dosage form suitable for oral administration or administration by injection.

8. The combination therapeutic agent of any one of claims 3 to 6, wherein the first pharmaceutical composition and the second pharmaceutical composition are separately formulated in dosage forms suitable for oral administration or administration by injection.

9. Use of the FGF21 Fc fusion protein of claim 1 and/or the GLP-1 Fc fusion protein of claim 2 in the preparation of a drug (preferably, the combination therapeutic agent of any one of claims 3 to 8) for the prevention or treatment of cardiovascular diseases and/or metabolic diseases.

10. The use of claim 9, wherein the cardiovascular diseases and/or metabolic diseases comprise, but are not limited to, obesity, hyperlipidemia, atherosclerosis, non-alcoholic fatty liver disease, diabetes, diabetic cardiomyopathy, coronary atherosclerotic heart disease, and other diseases associated with insulin resistance.
